# EUROPEAN PATENT APPLICATION

(11) **EP 1 466 620 A1**
(43) Date of publication of application: **13.10.2004**
(21) Application number: 03008435.4
(22) Date of filing: 11.04.2003
(51) Int. Cl.: A61K 38/24, G01N 33/50, A61P 25/00

(54) **Compounds modulating FSHR and therapeutic uses thereof**

(71) Applicant: AXXAM S.r.l., 20145 Milano (IT)
(72) Inventor: Tarroni, Paola, c/o Axxam S.r.l., 20132 Milano (IT); Lohmer, Stefan, c/o Axxam S.r.l., 20132 Milano (IT)
(74) Representative: Banfi, Paolo

(57) **Abstract**

The invention regards a method for screening compounds that interact with hFSHR (poly)peptides and therapeutic agents thereof, for use in the treatment of conditions affecting human central and peripheral nervous system, kidney, thymus, bronchus, trachea, aorta and prostate.

## Description

The present invention relates to the use of FSHR ligands for the treatment of conditions affecting human central and peripheral nervous system, kidney, thymus, bronchus, trachea, aorta and prostate. Also provided by the invention is a method of screening compounds useful for the treatment of the same diseases, which utilizes hFSHR polypeptides, cell preparations containing hFSHR polypeptides or cells expressing hFSHR polypeptides and containing receptor activity reporter system for in vitro assays.

### BACKGROUND OF THE INVENTION

This invention relates to human follicle stimulating hormone receptor and its expression studied by quantitative real time PCR and in situ hybridization techniques.

Follicle stimulating hormone (FSH) is a pituitary-derived heterodimeric glycoprotein hormone which shares structural similarities with luteinizing hormone (LH) and thyroid stimulating hormone (TSH), both of which are also produced in the pituitary gland, and chorionic gonadotropin (CG), which is produced in the placenta. The hormones are relatively large (28-38 kilodaltons) and are composed of a common alpha subunit non-covalently bound to a distinct beta subunit that confers receptor binding specificity.

Follicle-stimulating hormone (FSH) is essential for normal reproductive function in males and females (1).

The cellular receptors for glycoprotein hormones are known to be members of the G protein-coupled class of membrane-bound receptors which, when activated, stimulate an increase in the activity of adenylyl cyclase. This results in an increase in the level of the intracellular second messenger adenosine 3',5'-monophosphate (cAMP), which in turn causes increased steroid synthesis and secretion. Hydropathicity plots of the amino acid sequences of these receptors reveal three general domains: (a) a hydrophilic amino-terminal region, considered to be the amino-terminal extracellular domain, (b) seven hydrophobic segments of membrane-spanning length, considered to be the transmembrane domain, and (c) a carboxy-terminal region which contains potential phosphorylation sites (serine, threonine, and tyrosine residues), considered to be the carboxy-terminal intracellular or cytoplasmic domain. The glycoprotein hormone receptor family is distinguished from other G protein-coupled receptors, such as the beta 2-adrenergic, rhodopsin, and substance K receptors, by the large size of the hydrophilic amino-terminal domain, which is involved in hormone binding.

A cDNA encoding the FSH receptor (FSHR) has been isolated and sequenced by Minegish et al. (1). The deduced amino acid sequence of 678 residues contains 7 putative transmembrane segments and displays sequence similarity to G protein-coupled receptors. The 359-residue extracellular domain contains 4 N-linked glycosylation sites. While the protein is 89% identical overall with the previously cloned rat FSH receptor, the most highly conserved regions are the putative transmembrane segments, which show 95% similarity. Kelton et al. (2) also cloned the FSHR gene. Rousseau-Merck et al (3) used cDNA probes corresponding to the 5-prime and the 3-prime regions of the FSHR gene for chromosomal localization of the gene by in situ hybridization. They found that the location, 2p21-p16, is similar to that of the luteinizing hormone-choriogonadotropin receptor gene (LHCGR). Gromoll et al. (4) also mapped the FSHR gene to 2p21 by fluorescence in situ hybridization.

Gromoll et al. (5) determined that the FSHR gene contains 10 exons, spans 54 kb of genomic DNA, and encodes a 695-amino acid predicted protein. Exon 10 is the largest (1,234 bp) and contains the C-terminal part of the extracellular, transmembrane, and intracellular domains. Amino acid sequence similarity is high in comparison with the previously described rat gene (6), ranging from 82% in exon 9 to 100% in exon 3.

The FSH receptor is known to be expressed on testicular Sertoli cells and ovarian granulosa cells (7, 8).

FSH-beta (FSH beta) and FSH receptor (FSHR) knockout mice display impaired ovarian follicular development and infertility in females and small testes, oligospermia, and fertility in males. Humans with FSH beta gene mutations tend to have a more severe phenotype than those with FSHR gene mutations, although infertility and varying degrees of impaired sex steroid production occur in both types of mutations. Data from human and mouse mutations in the FSH beta and FSHR genes suggest that FSH is necessary for normal pubertal development and fertility in males and females (9).

Results from experiments using mouse models suggest that the role of follicle-stimulating hormone (FSH) in spermatogenesis is the regulation of Sertoli cell proliferation and, ultimately, the size and spermatogenic capacity of the testis. The regulation of the expression of the FSH receptor (FSHR) gene is very cell specific and plays an initial role in the ultimate response of the Sertoli cells to FSH (10).

The first direct evidence that granulosa cells express FSHR was obtained by autoradiographic localization of 125I-labelled FSH binding to immature rat ovarian sections (11). Radioligand-binding analysis confirmed the presence of specific, saturable, high-affinity FSH binding sites in granulosa cell membranes (12). With the isolation of cDNA clones encoding rat FSHR (13), the tissue distribution and developmental regulation of FSHR gene expression was finally mapped (14). That work confirmed gonad-specific expression of an abundant ~2.6 kb-sized FSHR mRNA and a less abundant ~5.0 kb transcript. In ovary, in situ hybridisation showed FSHR mRNA to be localised exclusively to the granulosa cells of healthy follicles, persisting throughout preovulatory follicular development.

The major related pharmaceutical product active on the FSHR is the recombinant hFSH that is used in clinical practice to treat infertile women and men with low or lacking naturally occurring FSH.

### DISCLOSURE OF THE INVENTION

The invention is based on the finding that FSHR is expressed in neurons present in the central and peripheral nervous system, as well as in the kidney, thymus, bronchus, trachea, aorta and prostate of humans.

The expression levels of FSH receptor have been studied in non diseased human tissues from various body districts, by means of quantitative real-time RT-PCR (TaqMan). The expression of FSH receptor in the human nervous system has also been investigated by radioactive in situ hybridization. It has been found that in normal individuals FSH receptor is surprisingly expressed not only in reproductive organs but also in different regions of the nervous system and in the above-indicated peripheral tissues. The expression in the nervous system is accountable to neurons, but not to glial or accessory cells. Positive signals were also found in spinal cord neurons and DRG neurons.

Surprisingly, rat and human profiling of FSH receptor in the nervous system resulted completely different being the mRNA encoding for the FSH receptor undetectable in rat tissues.

In a first embodiment, the invention provides the use of molecules modulating the activity of hFSH-receptor for the preparation of therapeutic agents for the treatment of diseases affecting human central and peripheral nervous system, bronchus, trachea, colon, thymus, kidney, testis, prostate, ovary, blood vessels. Molecules modulating the activity of hFSH-receptor can be selected from FSHR agonists and antagonists, in particular FSH or analogues or derivatives thereof.

According to a preferred embodiment, molecules modulating hFSHR activity are used in the treatment of conditions of the central nervous system which involve pain, anorexia, bulimia, Parkinson's diseases, psychotic and neurological disorders, anxiety, schizophrenia, manic depression, delirium, dementia, mental retardation and dyskinesias, Huntington's disease and Touret's syndrome.

In a further embodiment, the invention provides a method of screening compounds useful for the treatment of diseases affecting bronchus and trachea, colon, thymus, kidney, testis, prostate, ovary, blood vessels, central and peripheral nervous system, said method comprising a) contacting a hFSHR polypeptide with a test compound and b) detecting the binding of the polypeptide to the test compound. Compounds binding to hFSHR-polypeptides can be further screened for their ability to modulate the activity of the hFSHR polypeptides, in particular for their ability to increase or reduce the effects of FSHR ligands, such as FSH, analogues or derivatives thereof, on hFSHR polypeptides. Preferably the following effects mediated by FSHR are detected upon stimulation with appropriate ligands: i) modulation of phospholipase C or D activity; ii) cyclic AMP formation; iii) mobilization of intracellular calcium.

The screening methods can be carried out by means of high-throughput techniques, using cells suitably modified to express the relevant FSHR polypeptide and a suitable reporter system. Details on cell-based assays are provided below.

### DESCRIPTION OF THE DRAWINGS

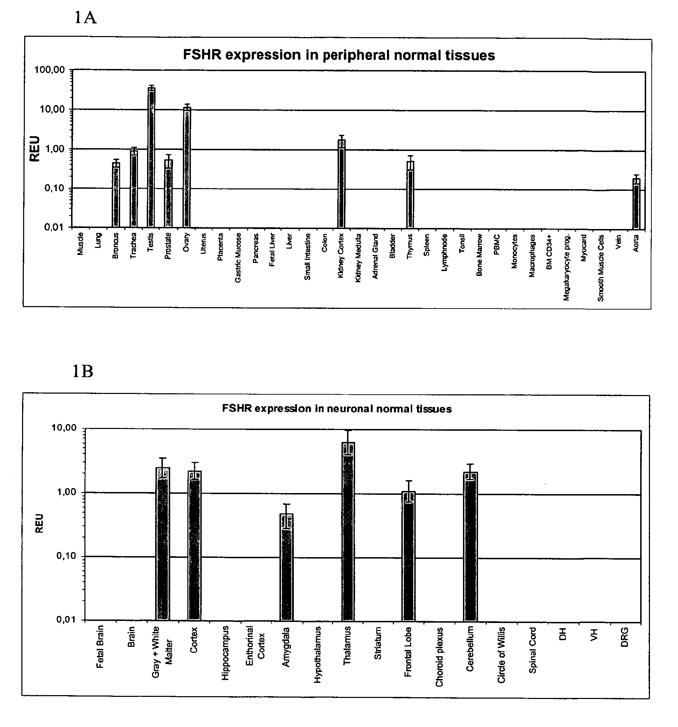

**FIG. 1** Expression profiling of mRNA of the FSH receptor in non diseased human tissues
   Relative FSHR gene expression levels in human tissues as listed in Table 1. A FAM- and MGBNFQ (Minor groove binder/Non-fluorescent quencher) -dual-labeled FSHR TaqMan "MGB" probe was used with primer set to examine mRNA levels in non diseased human tissues as described under the Materials and methods. The normalized mRNA levels are expressed as relative expression units (REU), i.e. ratio of FSHR transcript to 18S transcript. The REU value is the mean from two independent runs, each run with duplicate samples.
   Panel A demonstrate that in addition to expression in ovary and testis some specific FSHR mRNA expression is detectable in bronchus, trachea, prostate, kidney cortex, tymus and aorta.
   Panel B demonstrate that in the nervous system some specific FSHR mRNA expression is detectable in cortex, amygdale, thalamus, cerebellum.

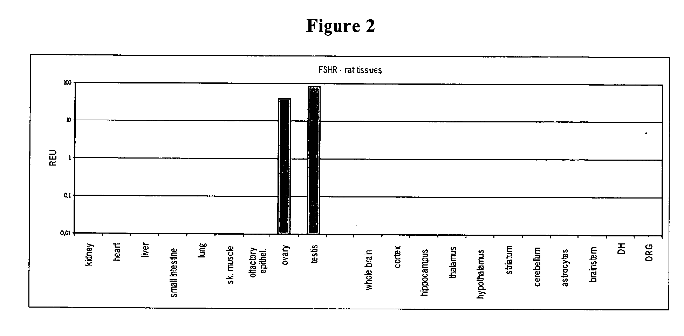

**FIG. 2** Expression profiling of mRNA of the FSH receptor in rat tissues. Relative FSHR gene expression levels in rat tissues as listed in Table 2. A FAM- and MGBNFQ (Minor groove binder/Non-fluorescent quencher) - dual-labeled FSHR TaqMan MGB probe was used with primer set to examine mRNA levels in non diseased rodent tissues as described under the Materials and methods. The normalized mRNA levels are expressed as relative expression units (REU), i.e. ratio of FSHR transcript to 18S transcript. The REU value is the mean from two independent runs, each run with duplicate samples. The picture demonstrate that in rat no detectable expression of FSHR mRNA is appreciable in tissues other than testis and ovary.
**FIG. 3** Human ovary tissue, in situ hybridization of hFSHR. AS - hybridization with anti sense probe - positive control. S- hybridization with sense probe - negative control. BF - Bright Field. DF - Dark Field.
**FIG. 4** Human spinal cord and DRG, in situ hybridization of hFSHR. BF - Bright Field. DF - Dark Field.
**FIG. 5** Human pons, cerebellum and nucleus dentatus, in situ hybridization of hFSHR. BF - Bright Field. DF - Dark Field.
**FIG. 6** Human substantia nigra and thalamus, in situ hybridization of hFSHR. BF - Bright Field. DF - Dark Field.
**FIG. 7** Human cortex and amigdala, in situ hybridization of hFSHR. BF - Bright Field. DF - Dark Field.
**FIG. 8** Human thalamus tissue, in situ hybridization of hFSHR. AS - hybridization with anti sense probe - positive control. S- hybridization with sense probe - negative control. BF - Bright Field. DF - Dark Field.
**FIG. 9** Stimulation of cAMP production (measured by luminescence) by human FSH in CHO cells transiently transfected with human FSH receptor cDNA. Transfected cells were treated increasing concentrations of human FSH for 4 hours at 37°C. Intracellular cAMP production was measured by luciferase activity. Each bar represents the mean of quadruplicate values.
**FIG. 10** Functional activation of human FSH receptor-Gα15 fusion in CHO cells (bioluminescence assay). Cells derived from the stably transfected clone K4 were stimulated with increasing concentrations of human FSH.

### DETAILED DESCRIPTION OF THE INVENTION

### Tissue-specific expression of FSHR

As a first step to establishing a role for *FSHR* in other disease states than reproductive disorders, expression profiling of the gene was done using real-time quantitative RT-PCR (TaqMan) on RNA samples isolated from a wide range of human tissues and cells. Total RNA samples were either purchased from commercial suppliers or purified in-house. RNA samples used for profiling are listed in Table 1.

The expression of *FSHR* in various human tissues is shown in Figure 2, A & B. By far the highest level of expression were found in testis and ovary but there was clearly detectable expression in other tissues like kidney. Detailed investigation of the expression of the receptor in various regions of adult brain revealed expression across the CNS, with cerebellum, cortex and thalamus exhibiting the highest level of expression (Figure 2B). The relative expression units (REU) values for the FSHR receptor mRNA are listed in Tab. 1.

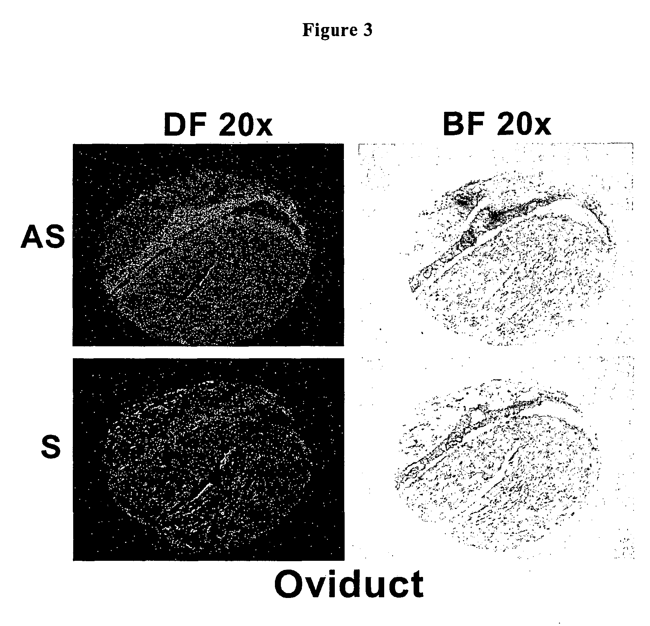

We have performed a similar study on samples derived from rat tissues listed in Tab. 2 in order to gain information on FSHR expression in the most diffused laboratory animal for in vivo models. As shown in Fig. 3 we have found expression of the mRNA encoding the receptor in testis and ovary as published and not in other tissues and in the nervous system. These data demonstrate that FSHR expression has species specific differences and this might account for expression in the human nervous system being previously undiscovered. The relative expression unit (REU) values for the rat FSHR receptor are listed in Tab. 2.

### Expression analysis of FSHR in human cell lines.

Expression of FSHR in a range of human cells lines (listed in Tab. 3) was also performed. Expression of specific FSHR mRNA in any of the cell sample tested proved to be undetectable (data not shown).

### In situ hybridization of FSHR

In order to proceed further in the characterization of FSHR in the human nervous system we have performed an expression study by *in situ* hybridization. By this techniques we were able to:
1) confirm specific expression in areas of the nervous system with clearly positive signal in TaqMan as cortex, thalamus, cerebellum;
2) find specific expression in areas with barely detectable or undetectable signal in TaqMan or where TaqMan was not performed as substantia nigra, pons, nucleus dentatus, hippocampus, amygdale, spinal cord and DRG,
3) characterize the cell type responsible for FSHR expression in the nervous system.

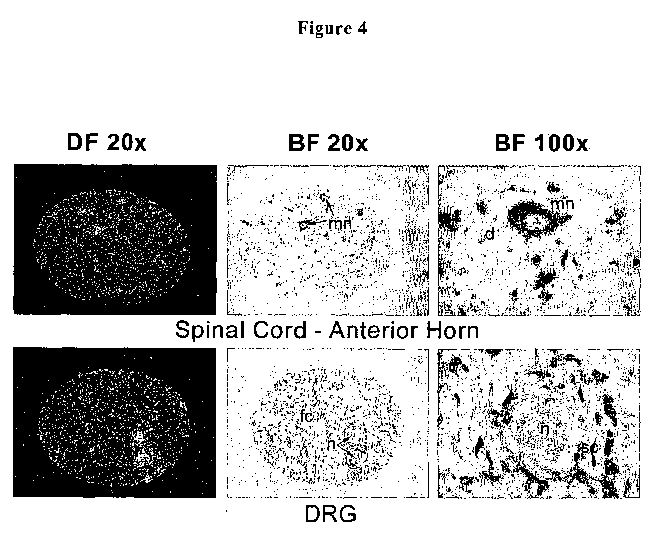

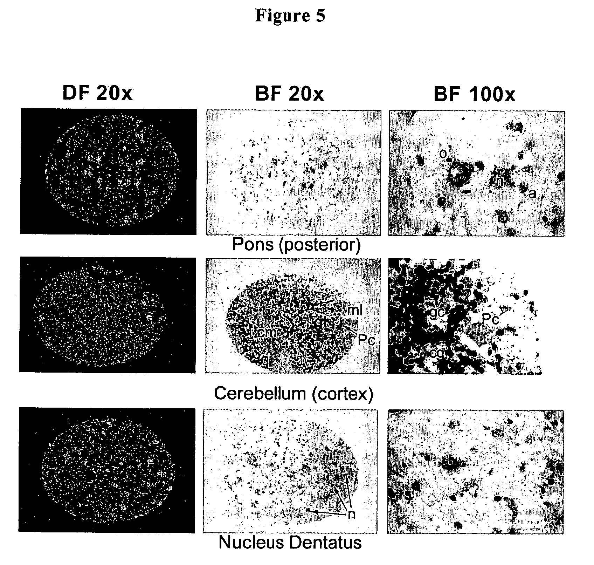

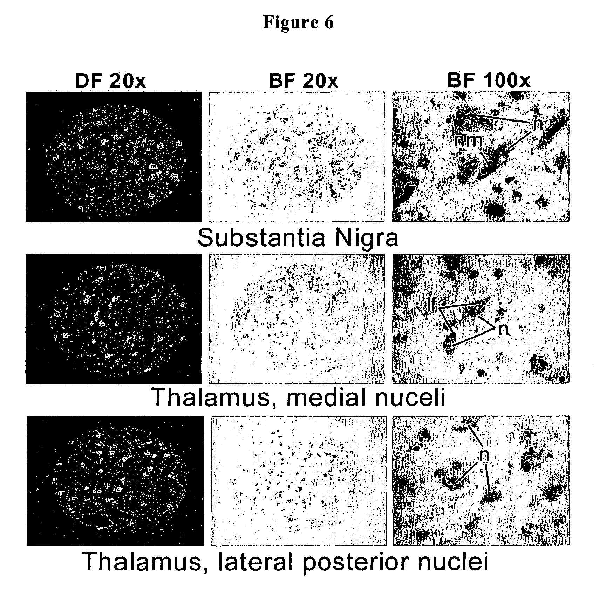

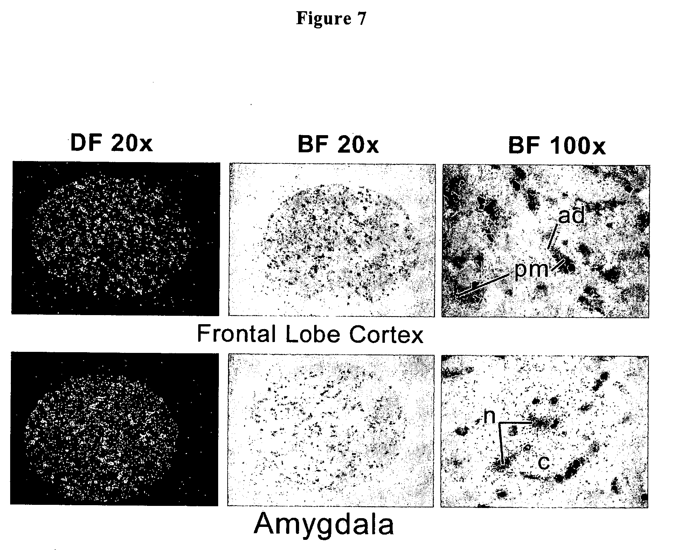

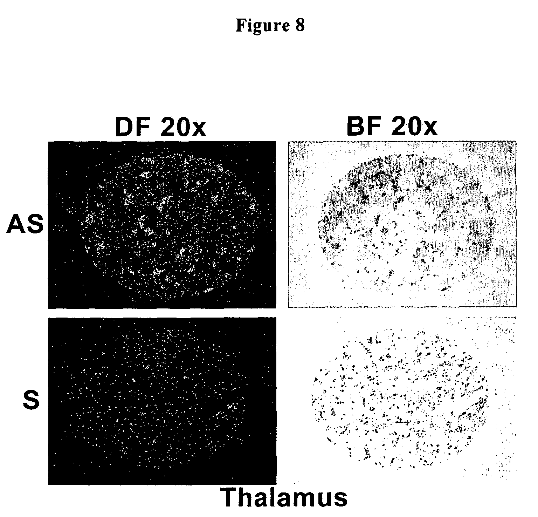

In general we have found a positive signal in "control" tissues such as ovary, where expression is well documented and it is described, as we found, in epithelia of the oviduct (Fig. 3). Then we have inquired expression in different region of the brain, spinal cord and DRG. We have found positive signals in the following sections, Spinal Cord, DRGs (Fig. 4), Pons, Cerebellum, Nucleus Dentatus (Fig. 5), Substantia Nigra, Thalamus (Fig. 6), Cortex and Amigdala (Fig. 7). Furthermore we have found that positive signal is due to expression in neurons, and not in other cells of the nervous system like glial cells. Appropriate negative controls were performed (Fig. 8). The following sections present a detailed description of the distributions of the FSHR.

### Thalamus

The Thalamus is the largest component of the diencephalon and is subdivided into the following major nuclear groups: Anterior, Medial, Lateral, Intralaminar and Reticular, Midline, Posterior.

We have examined section of the Medial Nuclear Group and of the Lateral posterior Nuclear group.

The medial nucleus belongs to a neural system concerned with affective behavior, memory and the integration of somatic and visceral activities.

The lateral nuclear group of the thalamus includes the lateral dorsal, lateral posterior and pulvinar nuclei. There is evidence that the pulvinar-lateral posterior complex plays a role in visual and speech mechanisms.

It has also been shown to play a role in pain mechanisms as lesions in the pulvinar nucleus have been effective in the treatment of intractable pain. Experimental studies have demonstrate connections between this complex and several cortical and sub cortical areas concerned with pain mechanisms.

We have observed expression of FSHR mRNA in neurons of the examined thalamic regions but not in glial cells.

### Cerebellum - cortex

The cerebellar cortex is made up of the following three layers: outer molecular layer, middle purkinjie cell layer, inner granule cell layer. Of the five cells types, distributed in the different cortical layers, the Purkinjie cell constitutes the principal neuron of the cerebellum, since it is the only cerebellar neuron that sends its axons outside the cerebellum. All the others cells are intrinsic neurons and establish connections within the cerebellum.

Containing more than half the neuron of the brain, the cerebellum is one of the busiest neuronal intersection in the brain, receiving input from and sending signal back to every major central nervous system. The three main sources of afferent are the spinal cord, vestibular system and cerebral cortex.

The cerebellum traditionally has been relegated a motor function. Asthenia, ataxia and atonia are synonymous of cerebellar disease; clinical and experimental studies confirmed a role for the cerebellum in control and integration of motor activity.

We have observed expression of FSHR mRNA in Purkinjie neurons of the cerebellar Cortex.

### Cerebellum - Nucleus Dentatus

The deep cerebellar nuclei are embedded in the white matter core of the cerebellum. There are four pairs of nuclei arranged from lateral to medial as follows: dentate, emboliform, globose and fastigi.

The dentate Nucleus is composed of multipolar neurons, it receives axons of Purkinj e cells located in the lateral part of the cerebellar hemispheres and collateral of climbing and mossy fibers. The Purkinje cell input is inhibitory, whereas the inputs from climbing and mossy fibers are excitatory to the dentate nucleus.

As in other nervous tissues studied we found strong levels of expression in all neurons of the ND. No expression has been detected in Oligodendroglial cells.

### Pons

The Pons is the part of the Brain stem that lies between the medulla oblongata caudally and the midbrain rostrally. The Dorsal Surface is covered by the cerebellum.

The functional significance of pontine connections is not known, but it may represent the anatomic substrate for the effect of emotion on motor function. The input from the associated cortices suggests a role for this fiber system in behavioral and cognitive processes.

The nucleus ceruleus contained in the Tegumentum is the source of noradrenergic innervations to most regions of the central nervous system. Cell loss in the nucleus is generalized in Parkinson's disease, whereas it is limited to the rostral portion of the nucleus, which projects mainly to the cerebral cortex, in Alzheimer's disease and Down syndrome.

As in others nervous tissues studied we found strong levels of expression in all neurons of the Pons (Tegumentum), motoneurons and sensitive neurons. No expression has been detected in glia cells.

### Substantia nigra

Substantia Nigra is a pigmented mass, belonging to the mesencephalon, of neurons sandwiched between cerebral peduncles and the tegumentum. The neuronal population of the substantia nigra consists of pigmented and non pigmented neurons. Pigmented neurons outnumber non pigmented neurons by two to one. The neurotransmitter in pigmented neurons is Dopamine. Non pigmented neurons are either cholinergic or GABAergic.

There is a characteristic pattern of neuronal loss in substantia nigra in different disease states. Both pigmented and non pigmented cells are lost in patients with Huntington's Chorea; only pigmented dopaminergic neurons are lost in Parkinson's disease.

The neuronal connectivity of the Substantia Nigra suggests an important role in the regulation of motor activity.

From the ISH experiments on Sustantia Nigra tissues we found similar levels of expression both in pigmented than in non pigmented neurons. No expression at all has been found in accessory cells (glia cells).

### Amygdale

The amygdalar nuclei, a major part of the limbic system, are located in the tip of the temporal lobe beneath the cortex. Several neurotransmitters have been demonstrated in the amygdale, including acetylcholine, GABA, noradrenalin, serotonin and dopamine.

The functions of the amygdale is somewhat elusive. The intricate neuronal connections of the amygdale make it difficult to ascribe an observed behavior purely to amygdale. The following manifestations however have been noted to occur after stimulation or ablation of the amygdale:
a) Autonomic Effects: Changes in heart rate, respiration, blood pressure and gastric motility. b) Orienting response, emotional behavior and food intake. c) Sexual activity: the amygdale contains the highest density of receptors of sex hormones.

We found strong levels of expression in all neurons, motoneurons and sensitive neurons. No expression has been detected in glia cells.

### Frontal lobe cortex

The prefrontal cortex refers to the area of the cortex comprising the pole of the frontal lobe. Motor responses are as a rule not elicited by stimulation of this area of the frontal lobe. The prefrontal cortex is well developed only in primates and especially so in humans; it is believed to play a role in affective behavior and judgment.

Lesions in prefrontal cortex result in inappropriate judgmental behavior and emotional liability. More dramatic effects are seen in bilateral lesions, such patients usually neglect their appearance, laugh or cry inappropriately, and have no appreciations of norms of social behavior and conduct.

We found strong levels of expression in pyramidal neurons.

### Spinal cord

In Cross sections SC is composed of a centrally placed butterfly or H shaped area of gray mattered surrounded by the white matter. The gray matter contains primarily the cells body of neuronal and glia. The white matter of the cord contains fiber tracts. The central gray matter is formed of dorsal (sensory), intermediate, and ventral (motor) horns. Each horn is formed of aggregates of neurons called nuclei.

The Dorsal Horn receives axons of the dorsal root ganglia via the dorsal root and contains cell clusters concerned with sensory function. The Intermediolateral Horn is limited to the thoracic and upper lumbar segments of the cord. It contains cell bodies of the sympathetic nervous system. The Ventral Horn contains multipolar motor neurons, axons of which comprise the major component of the ventral root.

Experiments studies have been performed on Spinal cord sections at thoracic level. We have found clear hybridization signals at level of the ventral horn. As in other nervous tissues studied we found strong levels of expression in neurons and not in glial cells (Astrocytes and Oligodendoglial) being, in this case, the positive cells motor neurons.

### DRG

The dorsal root ganglia are concerned with sensory reception and distribution. They receive stimulation from the external and internal environments at their distal ends and transmit nerve impulses to the central nervous system. The ganglion cells fall into two size groups. The smaller neurons have un-myelinated axons (sensory), whereas the larger cells have myelinated axons (motor). Each ganglion cell is surrounded by connective tissue and supporting cells (the perineuronal cells or capsule cells).

In situ hybridization has been performed on DRG coming from the thoracic tract. We found high levels of expression particularly in larger myelinated neurons with motor functions. No expression has been detected in satellite perineuronal cells and in surronding connective tissue.

### Therapeutic Indication

Drugs modulating the FSHR may therefore be used to treat and/or diagnose a variety of disorders that arise in the tissues in which it is expressed.

Disorders involving the bronchus and trachea include, but are not limited to, congenital anomalies; atelectasis; diseases of vascular origin, such as pulmonary congestion and edema, including hemodynamic pulmonary edema and edema caused by microvascular injury, adult respiratory distress syndrome (diffuse alveolar damage), pulmonary embolism, hemorrhage, and infarction, and pulmonary hypertension and vascular sclerosis; chronic obstructive pulmonary disease, such as emphysema, chronic bronchitis, bronchial asthma, and bronchiectasis; diffuse interstitial (infiltrative, restrictive) diseases, such as pneumoconioses, sarcoidosis, idiopathic pulmonary fibrosis, desquamative interstitial pneumonitis, hypersensitivity pneumonitis, pulmonary eosinophilia (pulmonary infiltration with eosinophilia), Bronchiolitis obliterans-organizing pneumonia, diffuse pulmonary hemorrhage syndromes, including Goodpasture syndrome, idiopathic pulmonary hemosiderosis and other hemorrhagic syndromes, pulmonary involvement in collagen vascular disorders, and pulmonary alveolar proteinosis; complications of therapies, such as drug-induced lung disease, radiation-induced lung disease, and lung transplantation; tumors, such as bronchogenic carcinoma, including paraneoplastic syndromes, bronchioloalveolar carcinoma, neuroendocrine tumors, such as bronchial carcinoid, miscellaneous tumors, and metastatic tumors; pathologies of the pleura, including inflammatory pleural effusions, noninflammatory pleural effusions, pneumothorax, and pleural tumors, including solitary fibrous tumors (pleural fibroma) and malignant mesothelioma.

Disorders involving the thymus include developmental disorders, such as DiGeorge syndrome with thymic hypoplasia or aplasia; thymic cysts; thymic hypoplasia, which involves the appearance of lymphoid follicles within the thymus, creating thymic follicular hyperplasia; and thymomas, including germ cell tumors, lynphomas, Hodgkin disease, and carcinoids. Thymomas can include benign or encapsulated thymoma, and malignant thymoma Type I (invasive thymoma) or Type II, designated thymic carcinoma.

Disorders involving the kidney include, but are not limited to, congenital anomalies including, but not limited to, cystic diseases of the kidney, that include but are not limited to, cystic renal dysplasia, autosomal dominant (adult) polycystic kidney disease, autosomal recessive (childhood) polycystic kidney disease, and cystic diseases of renal medulla, which include, but are not limited to, medullary sponge kidney, and nephronophthisis-uremic medullary cystic disease complex, acquired (dialysisassociated) cystic disease, such as simple cysts; glomerular diseases including pathologies of glomerular injury that include, but are not limited to, in situ immune complex deposition, that includes, but is not limited to, anti-GBM nephritis, Heymann nephritis, and antibodies against planted antigens, circulating immune complex nephritis, antibodies to glomerular cells, cell-mediated immunity in glomerulonephritis, activation of alternative complement pathway, epithelial cell injury, and pathologies involving mediators of glomerular injury including cellular and soluble mediators, acute glomerulonephritis, such as acute proliferative (poststreptococcal, postinfectious) glomerulonephritis, including but not limited to, poststreptococcal glomerulonephritis and nonstreptococcal acute glomerulonephritis, rapidly progressive (crescentic) glomerulonephritis, nephrotic syndrome, membranous glomerulonephritis (membranous nephropathy), minimal change disease (lipoid nephrosis), focal segmental glomerulosclerosis, membranoproliferative glomerulonephritis, IgA nephropathy (Berger disease), focal proliferative and necrotizing glomerulonephritis (focal glomerulonephritis), hereditary nephritis, including but not limited to, Alport syndrome and thin membrane disease (benign familial hematuria), chronic glomerulonephritis, glomerular lesions associated with systemic disease, including but not limited to, systemic lupus erythematosus, Henoch-Schonlein purpura, bacterial endocarditis, diabetic glomerulosclerosis, amyloidosis, fibrillary and immunotactoid glomerulonephritis, and other systemic disorders; diseases affecting tubules and interstitium, including acute tubular necrosis and tubulointerstitial nephritis, including but not limited to, pyelonephritis and urinary tract infection, acute pyelonephritis, chronic pyelonephritis and reflux nephropathy, and tubulointerstitial nephritis induced by drugs and toxins, including but not limited to, acute drug-induced interstitial nephritis, analgesic abuse nephropathy, nephropathy associated with nonsteroidal antiinflammatory drugs, and other tubulointerstitial diseases including, but not limited to, urate nephropathy, hypercalcemia and nephrocalcinosis, and multiple myeloma; diseases of blood vessels including benign nephrosclerosis, malignant hypertension and accelerated nephrosclerosis, renal artery stenosis, and thrombotic microangiopathies including, but not limited to, classic (childhood) hemolytic-uremic syndrome, adult hemolytic-uremic syndrome/thrombotic thrombocytopenic purpura, idiopathic HUS/TTP, and other vascular disorders including, but not limited to, atherosclerotic ischemic renal disease, atheroembolic renal disease, sickle cell disease nephropathy, diffuse cortical necrosis, and renal infarcts; urinary tract obstruction (obstructive uropathy); urolithiasis (renal calculi, stones); and tumors of the kidney including, but not limited to, benign tumors, such as renal papillary adenoma, renal fibroma or hamartoma (renomedullary interstitial cell tumor), angiomyolipoma, and oncocytoma, and malignant tumors, including renal cell carcinoma (hypernephroma, adenocarcinoma of kidney), which includes urothelial carcinomas of renal pelvis.

Disorders involving the prostate include, but are not limited to, inflammations, benign enlargement, for example, nodular hyperplasia (benign prostatic hypertrophy or hyperplasia), and tumors such as carcinoma.

Disorders involving the brain include, but are not limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states-global cerebral ischemia and focal cerebral ischemia-infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropodborne (Arbo) viral encephalitis, Herpes simplex virus Type 1, Herpes simplex virus, Type 2, Varicalla-zoster virus (Herpes zoster), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer disease and Pick disease, degenerative diseases of basal ganglia and brain stem, including Parkinsonism, idiopathic Parkinson disease (paralysis agitans), progressive supranuclear palsy, corticobasal degenration, multiple system atrophy, including striatonigral degenration, Shy-Drager syndrome, and olivopontocerebellar atrophy, and Huntington disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B1) deficiency and vitamin B12 deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Disorders involving blood vessels such as aorta include, but are not limited to, responses of vascular cell walls to injury, such as endothelial dysfunction and endothelial activation and intimal thickening; vascular diseases including, but not limited to, congenital anomalies, such as arteriovenous fistula, atherosclerosis, and hypertensive vascular disease, such as hypertension; inflammatory disease--the vasculitides, such as giant cell (temporal) arteritis, Takayasu arteritis, polyarteritis nodosa (classic), Kawasaki syndrome (mucocutaneous lymph node syndrome), microscopic polyanglitis (microscopic polyarteritis, hypersensitivity or leukocytoclastic anglitis), Wegener granulomatosis, thromboanglitis obliterans (Buerger disease), vasculitis associated with other disorders, and infectious arteritis; Raynaud disease; aneurysms and dissection, such as abdominal aortic aneurysms, syphilitic (luetic) aneurysms, and aortic dissection (dissecting hematoma); disorders of veins and lymphatics, such as varicose veins, thrombophlebitis and phlebothrombosis, obstruction of superior vena cava (superior vena cava syndrome), obstruction of inferior vena cava (inferior vena cava syndrome), and lymphangitis and lymphedema; tumors, including benign tumors and tumor-like conditions, such as hemangioma, lymphangioma, glomus tumor (glomangioma), vascular ectasias, and bacillary angiomatosis, and intermediate-grade (borderline low-grade malignant) tumors, such as Kaposi sarcoma and hemangloendothelioma, and malignant tumors, such as angiosarcoma and hemangiopericytoma; and pathology of therapeutic interventions in vascular disease, such as balloon angioplasty and related techniques and vascular replacement, such as coronary artery bypass graft surgery.

### Development of mammalian cell lines expressing hFSHR and a functional reporter system.

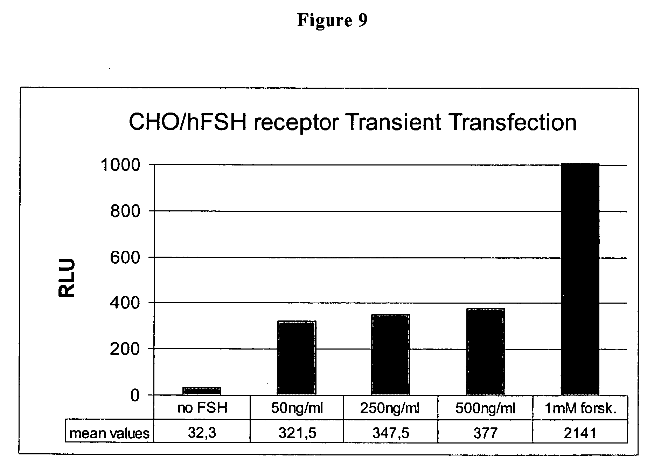

FSH receptor has been previously shown to be positively coupled to Gs, resulting in activation of adenylate cyclase and cAMP production (15). Functional activation of the receptor was evaluated by transient transfection in CHO cells. Treatment of transfected cells with the indicated hFSH concentrations (Fig. 9) induced dose-dependent increases in intracellular cAMP production. Basal value was calculated in transfected cells incubated 4 hours with vehicle only. For positive control, cells were incubated with 1 µM Forskolin. These results indicate that the recombinant hFSH receptor expressed in our cell system functionally couples with endogenous adenylyl cyclase.

In a different approach, in order to see if it is possible to switch the natural signal pathway of human FSH receptor from Gs to Gq, we have proceeded as follows.

We used CHO-Photin, an in-house produced CHO cell line expressing a Ca2+-sensitive Photo protein, named Photin (EP02023452), as reporter system for the detection of intracellular Ca2+ movements. In order to couple hFSH receptor to a signal transduction pathway leading to the release of intracellular free Ca2+, the hFSH receptor sequence was cloned without stop codon and in fusion at its 3' end with the Gα15sequence (16-17) and the outcoming construct was named hFSHR-Gα15-pcDNA3.

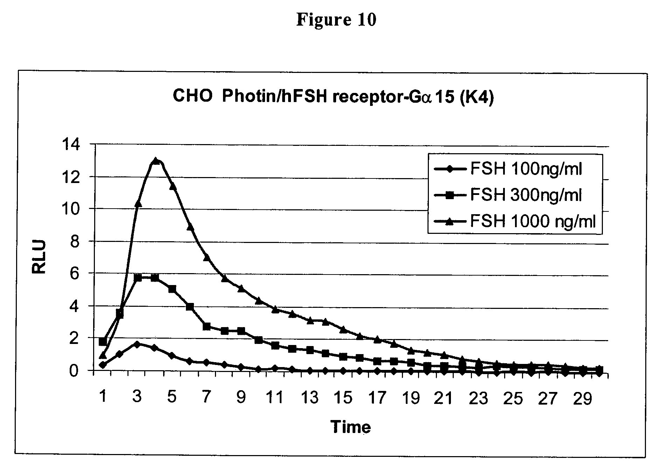

CHO-Photin cells were transfected by electroporation with hFSHR-Gα15-pcDNA3 construct and after antibiotic selection and limiting dilution, resistant stably transfected clones were tested for intracellular Ca2+movements after stimulation with FSH. The best responding clones were picked up, expanded and re-tested using different concentrations of hFSH. Among these clones, K4 was found to express hFSH receptor and responded to hFSH in a dose-dependent way (Fig. 10).

### Expression of recombinant FSHR

To express the human FSHR polypeptide, an FSHR polynucleotide can be inserted into an expression vector which contains the necessary elements for the transcription and translation of the inserted coding sequence. The DNA which is used to make expression vectors may be genomic DNA or cDNA encoding human FSH receptor, and may contain regions which enhance expression, such as introns, promoters, enhancers, etc. The DNA may be readily modified by substitution, deletion or insertion of nucleotides (e.g. by site-specific mutagenesis) that do not adversely affect the biological or FSH-binding activity of the expressed protein. For example, conservative substitutions (mutations) which alter from one to ten amino acids may be made without adversely affecting the overall structure and activity of the expressed protein (mutein). Likewise, certain portions of the DNA, such as those portions which code for the cytoplasmic and/or transmembrane domains, may be deleted so that only a fragment, such as the soluble extra cellular domain, of the protein is expressed. The human FSH receptor or FSH-binding fragment or mutant thereof may also be expressed as a fusion protein. One such fusion protein would include a polypeptide at the carboxy-terminus which would confer characteristics which would facilitate purification of the protein, or immobilization of the purified protein on a solid substrate for use in FSH assays or FSH purification protocols. Another such fusion protein would include a cleavable polypeptide at the amino-terminus which would facilitate expression.

Methods which are well known to those skilled in the art can be used to construct expression vectors containing sequences encoding FSHR polypeptides and appropriate transcriptional and translational control elements. These methods include in vitro recombinant DNA techniques, synthetic techniques, and in vivo genetic recombination.

Such techniques are described, for example, in Sambrook et al., 2000 (18) and in Ausubel 1988 (19).

A variety of expression vector/host systems can be utilized to contain and express sequences encoding an FSHR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors, plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those non-translated regions of the vector -enhancers, promoters, 5'and 3'untranslated regions- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (LifeTechnologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding an FSHR polypeptide, vectors based on SV40 or EBV can be used with an appropriate selectable marker.

If plant expression vectors are used, the expression of sequences encoding FSHR polypeptides can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used (20). These constructs can be introduced into plant cells by direct DNA transformation or by pathogen-mediated transfection. Such techniques are described in a number of generally available reviews.

An insect system also can be used to express an FSHR polypeptide. For example, in one such system Autographa califomica nuclear polyhedrosis virus (AcNPV) is used as a vector to express foreign genes in Spodopteraftugiperda cells or in Trichoplusia larvae. Sequences encoding FSHR polypeptides can be cloned into a non-essential region of the virus, such as the polyhedrin gene, and placed under control of the polyhedrin promoter. Successful insertion of FSHR polypeptides will render the polyhedrin gene inactive and produce recombinant virus lacking coat protein. The recombinant viruses can then be used to infect S. frugiperda cells or Trichoplusia larvae in which FSHR polypeptides can be expressed (21).

If mammalian host cells are used a number of viral-based expression systems can be used to express FSHR polypeptides. For example, if an adenovirus is used as an expression vector, sequences encoding FSHR polypeptides can be ligated into an adenovirus transcription/translation complex comprising the late promoter and tripartite leader sequence. Insertion in a non-essential E1 or E3 region of the viral genome can be used to obtain a viable virus which is capable of expressing an FSHR polypeptide in infected host cells (22). If desired, transcription enhancers, such as the Rous sarcoma virus (RSV) enhancer, can be used to increase expression in mammalian host cells.

Specific initiation signals also can be used to achieve more efficient translation of sequences encoding FSHR polypeptides. Such signals include the ATG initiation codon and adjacent sequences. In cases where sequences encoding an FSHR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

The efficiency of expression can be enhanced by the inclusion of enhancers which are appropriate for the particular cell system which is used (23).

A mammalian host cell strain can be chosen for its ability to modulate the expression of the inserted sequences or to process the expressed FSHR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function.

Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express FSHR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced FSHR sequences. Resistant clones of stably transformed cells can be proliferated using tissue culture techniques appropriate to the cell type.

Any number of selection systems can be used to recover transformed cell lines. These include, but are not limited to, the herpes simplex virus thymidine kinase (24) and adenine phosphoribosyltransferase (25) genes which can be employed in tk or aprt cells, respectively. Also, antimetabolite, antibiotic, or herbicide resistance can be used as the basis for selection. For example, dhfr confers resistance to methotrexate (26), npt confers resistance to the aminoglycosides, neomycin and G-418 (27), and als and pat confer resistance to chlorsulfuron and phosphinotricin acetyltransferase, respectively. Additional selectable genes have been described. For example, trpB allows cells to utilize indole in place of tryptophan, or hisD, which allows cells to utilize histinol in place of histidine (28). Visible markers such as anthocyanins, -glucuronidase and its substrate GUS, and luciferase and its substrate luciferin, can be used to identify transformants and to quantify the amount of transient or stable protein expression attributable to a specific vector system (29).

### Production of Altered FSHR Polypeptides

As will be understood by those skilled in the art, it may be advantageous to produce FSHR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence. The nucleotide sequences of the human FSHR can be engineered using methods generally known in the art to alter FSHR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

### Screening Methods

The invention provides assays for screening test compounds which bind to or modulate the activity of an FSHR polypeptide or an FSHR polynucleotide. A test compound preferably binds to an FSHR polypeptide or polynucleotide. More preferably, a test compound decreases or increases the effect of FSH or FHS analogs as mediated via human FSHR by at least about 10, preferably about 50, more preferably about 75, 90, or 100% relative to the absence of the test compound.

### Test Compounds

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds (30).

Methods for the synthesis of molecular libraries are well known in the art (31, 32). Libraries of compounds can be presented in solution (33), beads (34) or chips (35).

### High Throughput Screening

Test compounds can be screened for the ability to bind to FSHR polypeptides or to affect FSHR activity or FSHR gene expression using high throughput screening. Using high throughput screening, many discrete compounds can be tested in parallel so that large numbers of test compounds can be quickly screened. The most widely established techniques utilize 96-, 384- and 1536-well microtiter plates. The wells of the microtiter plates typically require assay volumes that range from 2 to 500 µl. In addition to the plates, many instruments, materials, pipettors, robotics, plate washers, and plate readers are commercially available to fit the 96-, 384- and 1536-well format.

Alternatively "free format assays" or assays that have no physical barrier between samples, can be used.

### Functional Assay

Test compounds can be tested for the ability to increase or decrease a biological effect of an FSHR polypeptide. Such biological effects can be determined using the functional assays described below. Functional assays can be carried out after contacting either a purified FSHR polypeptide, a cell membrane preparation, or an intact cell with a test compound. A test compound which decreases a functional activity of an FSHR by at least about 10, preferably about 50, more preferably about 75, 90, or 100% is identified as a potential agent for decreasing FSHR activity. A test compound which increases FSHR activity by at least about 10, preferably about 50, more preferably about 75, 90, or 100% is identified as a potential agent for increasing FSHR activity.

One such screening procedure involves the use of cells which express a human FSHR polypeptide (for example, transfected CHO cells) in a system which measures intracellular second messengers changes caused by receptor activation. For example, test compounds may be contacted with a cell which expresses a human FSHR polypeptide and a second messenger response (e.g., cAMP formation, intracellular calcium), can be measured, in direct or indirect way, to determine whether the test compound activates or inhibits the receptor.

For example, receptor-mediated stimulation of cAMP formation can be assayed in host cells which express human FSHR. Cells are plated in multi-well plates and incubated in Dulbecco's phosphate buffered saline (PBS) supplemented with 10 mM HEPES, 5 mM theophylline, 2 µg/ml aprotinin, 0.5 mg/ml leupeptin, and 10 µg/ml phosphoramidon for 20 minutes at 37°C in 5% C02. A test compound is added and incubated for an additional 10 minutes at 37°C. The medium is aspirated, and the reaction is stopped by the addition of 100 mM HCl. The plates are stored at 4°C for 15 minutes. cAMP content in the stopping solution is measured by radioimmunoassay.

Radioactivity is quantified using a gamma counter equipped with data reduction software. A test compound which decreases radioactivity of a well relative to radioactivity of a well in the absence of the test compound is identified as a potential inhibitor of cAMP formation. A test compound which increases radioactivity of a well relative to radioactivity of a well in the absence of the test compound is identified as a potential enhancer of cAMP formation.

Alternatively, a reporter gene such as luciferase, which may or may not be secreted by the cell, can be operatively linked to a cAMP response element, and increases in cAMP levels may be indirectly measured by a non-radioactive method, such as bioluminescence.

Intracellular free calcium concentration can be measured by microspectrofluorometry using the fluorescent indicator dye Fura-2/AM (36) or any other fluorescent indicator. Stably transfected cells are seeded onto a 35 mm culture dish containing a glass coverslip insert. Cells are washed with HBS, incubated with a test compound, and loaded with 100 µl of Fura-2/AM (10 µM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

Alternatively, a reporter such as a calcium sensitive luminescent protein, which may or may not be secreted by the cell or be located to any convenient cell compartment (cell membrane, mitochondrion, nucleus endoplasmic reticulum or cytosol), can be operatively linked calcium influxs or intracellular releases, and increases in intracellular calcium levels may be indirectly measured by a non-radioactive method, such as bioluminescence.

Another such screening technique involves introducing RNA encoding a human FSHR polypeptide into Xenopus oocytes to transiently express the receptor. The injected oocytes can then be contacted with the receptor ligand and a test compound to be screened, followed by detection of inhibition or activation of a reporter signal in the case of screening for test compounds which are thought to inhibit activation of the receptor.

### MATERIALS AND METHODS

***RNA purification.*** Total RNAs from the in-house samples detailed in Tables 1, 2 and 3 were isolated using TRIzol (Life Technologies) extraction according to the manufacturer's protocols. The concentration of purified RNA was determined spectrophotometrically by measurement of absorbance at 260 nm.

***cDNA preparation.*** Fifty micrograms of each RNA listed in Tables 1, 2 and 3, were treated with DNase according to the protocol for the kit DNA-free purchased from Ambion (Texas). The samples starting volumes was 50 µL, and all volumes to be added were adjusted accordingly.

After spectrophotometric quantification, each sample was reverse transcribed with the "SuperScript First-Strand Synthesis System for RT-PCR" kit (Invitrogen, CA). The concentration of RNA in the reaction was 140 ng/µL and reverse transcription was done with random hexamer primers at a concentration of 250 ng/µL.

### Real-time quantitative RT-PCR.

This technique is a development of the kinetic analysis of PCR first described by Higuchi et al. (37, 38). The principle is that at any given cycle within the exponential phase of PCR, the amount of product is proportional to the initial number of template copies. PCR amplification is performed in the presence of an oligonucleotide probe (TaqMan probe) that is complementary to the target sequence and labelled with a fluorescent reporter dye and a quencher dye. During the extension phase of PCR the probe is cleaved by the 5'-3' endonuclease activity of Taq DNA polymerase, releasing the fluorophore from the effect of the quenching dye (39). Because the fluorescence emission increases in direct proportion to the amount of the specific amplified product, the exponential growth phase of PCR product can be detected and used to determine the initial template concentration (40, 41). In addition, simultaneous amplification of an endogenous control can be performed to standardize the amount of sample RNA used in the reaction. In such experiments the control of choice is 18S ribosomal RNA. Reporter dyes with differing emission spectra are available, which enable the target and the endogenous control to be independently quantified in the same tube.

***Expression analysis:*** Specific primers and probe were designed according to the recommendations of Applied Biosystems and are listed below:
1. for the human FSHR transcript: 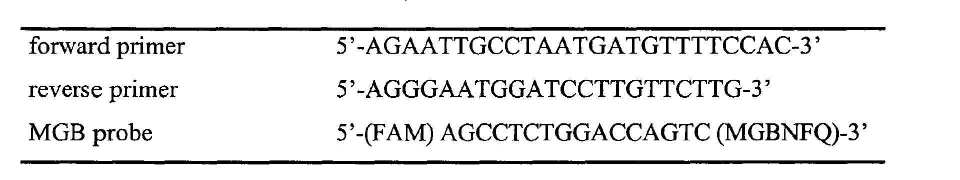 where FAM = 6-carboxy-fluorescein and MGBNFQ = Minor groove binder/Non-fluorescent quencher.
2. for the rat FSHR transcript: 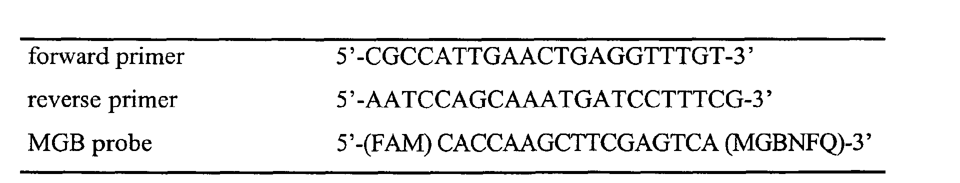
where FAM = 6-carboxy-fluorescein and MGBNFQ = Minor groove binder/Non-fluorescent quencher.

Quantitative PCR was performed with 10ng of reverse transcribed RNA from each sample. Each determination was done in duplicate, and the quantification was performed twice.

Total cDNA content was normalized by simultaneous quantification (multiplex PCR) of the 18S ribosomal RNA by using a Pre-Developed TaqMan Assay Reagents (PDAR) Control Kit (Applied Biosystems, CA). The assay reaction mix for human FSHR trasncript quantification was as follows:

| | Final conc. |
|---|---|
| TaqMan Universal PCR Master Mix (2x) | 1x |
| (Applied Biosystems, CA) | |
| PDAR control - 18S RNA (20x) | 1x |
| Forward primer | 300 nM |
| Reverse primer | 900 nM |
| Probe | 200 nM |
| cDNA | 10 ng |
| Water | to 25 µL |

The reaction for the rat FSHR transcript quantification was exactly the same, with the exception that the forward primer was used at a concentration of 900 nM.

For PCR, the following steps were carried out once: pre PCR, 2 minutes at 50°C, and 10 minutes at 95°C. The following steps were carried out 40 times: denaturation, 15s at 95°C; annealing/extension, 60s at 60°C.

All experiments were performed on an ABI Prism 7700 Sequence Detector (Applied Biosystems, Foster City, CA). At the end of the run, fluorescence data acquired during PCR were processed as described in the ABI Prism 7700 user's manual in order to achieve better background subtraction as well as signal linearity with the starting target quantity.

### In situ hybridization

### Probe labeling:

We used Riboprobes transcribed with high efficiency and specificity with an high percentage of full-length material than alkaline hydrolyzed to approx. 100bp fragments for better accession to tissue sections. To avoid sticking of 35S to plastic surfaces, 10 mM dithiothreithol (DTT) must be present in solutions. For RNAse free conditions we used only DEPC-treated solutions.

Labeling reaction mixes were prepared on ice according to following scheme.

| Basic Mix: | | | |
|---|---|---|---|
| reagent | volume x 4 reactions | volume x 6 reactions | volume x 10 reactions |
| 10x transcription buffer | 7.5 µl | 10.5 µl | 18 µl |
| ATP-CTP-GTP (10 mM each) | 11.25 µl | 15.75 µl | 27 µl |
| RNase inhibitor (40U/ µl) | 3.75 µl | 5.25 µl | 9 µl |
| DTT 0.1M | 7.5 µl | 10.5 µl | 18 µl |

| Reaction Mix: | |
|---|---|
| Reagent | Volume |
| H₂O* | - |
| Linearized plasmid DNA* | - |
| Basic Mix | 6 µl |
| 35S-UTP | 5 µl |
| RNA pol (T3, T7 or SP6) | 0.75 µl |

| | |
|---|---|
| * DNA plus water volume is 3.25 µl/reaction. Quantity of linearized plasmid: 1 µg/reaction. | |

Reaction mixes were incubated for 1 hr at 37°C. 0.5 µl DNase I were added per reaction, than incubated 15' at 37°C.

The following yeast tRNA solution was prepared and added 25 µl per labeling reaction.

| Yeast tRNA solution | | | |
|---|---|---|---|
| Reagent | Volume - 4 reactions | Volume - 6 reactions | Volume - 10 reactions |
| H2O (DEPC) | 110 µl | 154 µl | 264 µl |
| tRNA (10 mg/ml) | 15 µl | 21 µl | 36 µl |

Immediately 40 µl/reaction of alkaline solution A were added.

| Alkaline solution A: | | | |
|---|---|---|---|
| Reagent | Volume - 4 reactions | Volume - 6 reactions | Volume - 10 reactions |
| NaHCO3 1M | 16 µl | 24 µl | 48 µl |
| Na2CO3 1M | 24 µl | 36 µl | 72 µl |
| H2O | 158 µl | 237 µl | 474 µl |
| DTT (1M) | 2 µl | 3 µl | 6 µl |

Reactions were incubated at 60°C for time depending on transcribed probe's length (100-200 nt = 20'; 200-300 nt = 45'; 600 and more nt. = 60')

40 µl/reaction of alkaline solution B were added.

| Alkaline solution B: | |
|---|---|
| Reagent | 4-10 react |
| Na Acetate 1M | 100 µl |
| Glacial acetic acid | 5 µl |
| DTT1M | 5 µl |
| H2O | 390 µl |

For purification the mix was loaded on top of Sephadex G50 column (Roche Biochemicals) following purchaser instruction. Eluted probe fas EtOH precipitated following standard procedure.

In brief: 35 µl DTT/NH4Ac (DTT 1M 22 µl, Ammonium acetate 6M 396 µl) and than 250 µl ETOH were added to 100 µl of probe. Probes were incubated for 1.5 hrs or more at -20°C, cf. in eppendorf minifuge at +4°C for 20'. Then 500 µl of 75% ETOH in H2O-DEPC were added. Vortex briefly, centrifuge 10' at 4°C. Dry pellets at RT for 15-20'.

Probes were resuspended in 50 µl of 20 mM DTT.
2 µl were used for activity counting.

### Slide prehybridization and hybridization

### General notes

Material: 6 µm sections of 4% PFA-fixed paraffin-embedded tissue on Superfrost (+) slides (Menzel, Germany). All glassware should be sterilized, working area, pipettes etc. should be treated with commercial RNase killer (e.g. RNase Away).Unless stated otherwise, all the steps are carried out at RT Day 1

### Pretreatment of sections

1) Rehydration : 3x xylene, 100%, 96%, 85%, 70%, 50% ETOH 3' each
2) Removal of traces of ETOH: 5' Saline, 5' PBS
3) Post fixation in 4% PFA in PBS, 20'
4) 2x5' in PBS
5) Proteinase K treatment, 7.5'
6) PBS 5'
7) Dip into H20 DEPC
8) Acetylation: dip slides into stirred triethanolamine solution, add 0.25% of acetic anhydride, 15'
9) PBS 5'
10) Saline 5'
11) Dehydration: 30, 50, 70, 85, 96, 100, 100% ETOH (2' each, except 70% ETOH, in which the slides must be incubated for 10' to avoid salt deposition
12) Air dry use on the in the same day for hybridization

### Hybridization

1) Denature the probe in hybridization mix at 80°C for 2', cool the mix down on ice
2) Place 80-120 µl per slide, cover the slides with parafilm coverslips
3) Place the slides horizontally in humid chamber for 16-18 hrs Day 2

### High stringency washes

NB: all washes are to be carried out in clear jars, labeled "RNase". Estimated need of solution per jar: 200 ml. Mercaptoethanol to be used under chemical hood only.
1) Remove the cover slips in cover slip removal solution at RT
2) High stringency wash I, 30' 65°C
3) Washing solution II, 1x RT and 3x 37°C 10' each wash
4) Washing solution II + RNase A (final concentration 20 µg/ml), 1 hr 37°C
5) Washing solution II, 37°C 15'
6) High stringency washing solution, 65°C, 30'
7) 2xSSC, 15' RT
8) 0.1xSSC, 15' RT
9) Dehydration of sections: quick rinses (ca. 30") in 30, 60, 80, 96% ETOH containing 0.3M Na-Acetate, and 2x absolute ETOH
10) Air dry sections, attach the sections with tape to paper and expose to x-ray film ON

### Solutions for in situ slide treatments

4% Paraformaldehyde in PBS, 400 ml
- Weight 16 gr of PFA under the hood
- Add PFA to 350 ml of sterile PBS-DEPC
- Keep on waterbath at 65°C with vigorous shaking every 5' until the solution becomes completely clear
- Cool the solution down to RT
- Add PBS to 400 ml
   Saline, 0.85% NaCl, 400 ml
- 11.6 ml of 5M NaCl (2.9 ml per 100 ml)
- H20-DEPC till 400 ml
   Proteinase K, 20 µg/ml
- 20 ml of 1M Tris (pH 8.0)
- 4 ml 0.5M EDTA (pH 8.0)
- H2O-DEPC to 400 ml
- 400 µl of Proteinase K (stock 20 mg/ml)
   Triethanolamine (0.1M), 500 ml
- 6.65 ml of triethanolamine
- H2O-DEPC to 450 ml
- Adjust pH to 8.0 with 18.5% HCl, adjust volume to 500 ml with H2O-DEPC
Hybridization chamber solution, 50 ml
- Formamide 25 ml
- 5M NaCl, 3 ml
- 1M Na-Phosphate buffer (pH 8.0) 0.5 ml
- H2O to 50 ml

β-mercaptoethanol dilutions table (in wash solutions)

Final volume of solution (ml) Volume of β-mercaptoethanol (ml)
200 0.28
250 0.35
500 0.70
1000 1.40

Coverslips removal solution (0.5xSSC, 0.141% β-mercaptoethanol), 250 ml
- 62.5 ml of 20x SSC
- 0.35 ml of β-mercaptoethanol
- volume to 250 ml with water

High stringency washing solution (50% Formamide, 2xSSC, 0.141% β-mercaptoethanol), 400 ml per 1 set of slides
- 200 ml formamide
- 40 ml 20x SSC
- 0.56 ml β-mercaptoethanol
- volume to 400 ml with H2O

Washing solution II (0.5M NaCl, 10 mM Tris, 5 mM EDTA), 1000 ml per a set of slides
- 100 ml of 5m NaCl
- 10 ml of 1M Tris buffer (pH 8.0)
- 10 ml of 0.5M EDTA (pH 8.0)
- volume to 100 ml with water

### Hybridization mixture

Ratio in the hybridization mixture (HM): Basic Mix /probe= 88/12

| Basic mixture: | | | | |
|---|---|---|---|---|
| [Reagent] _{Cf} | Stock | vol_{f} in1 ml of HM | vol_{f} in 2 ml of HM | vol_{f} in 5 ml of HM |
| 50% formamide | 100% | 500 µl | 1000 µl | 2500 µl |
| 20mM Tris (pH8.0) | 1M | 20 µl | 40 µl | 100 µl |
| 5mM EDTA (pH8.0) | 0.5M | 10 µl | 20 µl | 50 µl |
| 1x Denhardt's sol | 50x | 20 µl | 40 µl | 100 µl |
| 10 mM DTT | 1M | 10 µl | 20 µl | 50 µl |
| 0.5 mg/ml yeast tRNA | 10 mg/ml | 50 µl | 100 µl | 250 µl |
| 10mM Na-Phosphate | 1M | 10 µl | 20 µl | 50 µl |
| buffer (pH 7.5) | | | | |
| 0.3M NaCl | 5M | 60µl | 120 µl | 300 µl |
| 10% Na Dextr SO4 | 50% | 200 µl | 400 µl | 1000 µl |
| H2O+labelled probe | - | 120 µl | 240 µl | 600 µ |

### Dipping, exposure and development of slides for in situ autoradiography

### Dipping in emulsion

It is necessary to have a verified, high quality dark-room (DO NOT USE ANY KIND OF SAFETY LAMP); Light microscopy autoradiography emulsion (Amersham RPN40) and waterbath without light diodes, set at 42°C.

Procedure (alls steps in the dark):
1) Melt the emulsion at 42°C (15-30'), combine the emulsion with prewarmed deionized water (20 ml+20 ml) in the dark
2) Dip the slides into emulsion
3) Let the excess of emulsion to drain away
4) Dry slides at RT for 2-4 hrs
5) Expose slides with silica gel at 4°C in boxes wrapped in aluminum foil

### Development of slides

It is necessary to have a developer D19 (Kodak): 170 g/l of powder dissolved in deionized water at 35°C at stirring, wrap the bottle in foil, cool to 15-16°C. Kodak Sodium fix (CAT 5221338): 150 g/l of powder dissolved in deionized water at RT. Jars/racks for slides

Procedure:
1) Warm the slides to RT (Take out from refrigerator 2 hrs before development)
2) Develop the slides for 3' in D 19 (15-17°C)
3) Rinse slides in deionized water (30')
4) Fix for 5' at RT
5) Rinse thoroughly in running tap water
6) Remove emulsion from the back of the slides
7) Counterstain, mount in DPX or analogous mounting media

### Cell culture and transient transfections

CHO cells stably transfected with a Luciferse-cAMP sensitive reporter system, were maintained at 37°C in DMEM/F12 medium with Glutamax-I supplemented with 10% FBS (GIBCO), and 100 U/ml penicillin/10 µg/ml streptomycin in a humified atmosphere containing 5% CO2.

FSHR-pCDNA3 plasmid, containing the complete coding sequence of the human FSHR (NM_000145), inserted in the KpnI-XhoI sites of the pCDNA3 MCS, was transiently transfected in the cells, seeded in 96 well plates, at 50-70% confluent, using FUGENE reagent (Roche) and following the manufacturer's instructions.

### Adenylate cyclase activity assay

24 h after transfection medium was removed and cells were incubated with different concentrations of FSH for 4 h at 37°C. Cells were then lysed and tested. Intracellular cAMP levels were determined by luminescence assay using a Berthold Luminometer. Each hormone concentration was assayed in quadruplicate.

### Stable transfections and intracellular calcium measurement

CHO cells stably expressing a Ca2+-sensitive photoprotein were transfected with FSHR-pcDNA3 vector, using Fugene reagent (Roche) and following the manufacturer's instruction. Cells were selected using standard culture medium supplemented with 2 mg/ml of G418 for two weeks. Resistant clones were pooled and subcloned by limiting dilution in 96 well plates to obtain individual clones. Two weeks later plates were duplicated and prepared to be used in the bioluminescence functional assay. Briefly, medium was removed and cells were incubated in standard tyrode solution (with 2 mM Ca2+) + 5 µg/ml coelenterazine for 3 h at 37°C. Plates were then loaded into the Luminoskan RS plate-reading luminometer (Labsystems, Needham Heights, MA), and wells were tested sequentially. 50 µl of tyrode containing 2X human FSH (CALBIOCHEM, cat. No. 869001) was injected in each well and light emission was recorded over 30 s.

### TABLES

**Tab. 1**

| human non pathological tissues | | | |
|---|---|---|---|
| **Tissue** | REU | **Tissue** | REU |
| **Musle** | 0,00 | **Macrophages** | 0,00 |
| **Lung** | 0,00 | **BM CD34+** | 0,00 |
| **Bronchus** | 0,44 | **Megakaryocyte prog.** | 0,00 |
| **Trachea** | 0,23 | **Myocard** | 0,00 |
| **Testis** | 35,11 | **Smooth Muscle Cells** | 0,00 |
| **Prostate** | 0,25 | **Vein** | 0,00 |
| **Ovary** | 11,37 | **Aorta** | 0,20 |
| **Uterus** | 0,00 | **Foetal Brain** | 0,00 |
| **Placenta** | 0,00 | **Brain** | 0,00 |
| **Gastric Mucose** | 0,00 | **Gray + White Matter** | 2,48 |
| **Pancreas** | 0,00 | **Cortex** | 2,21 |
| **Foetal Liver** | 0,00 | **Hippocampus** | 0,00 |
| **Liver** | 0,00 | **Enthorinal Cortex** | 0,00 |
| **Small Intestine** | 0,00 | **Amygdala** | 0,48 |
| **Colon** | 0,00 | **Hypothalamus** | 0,00 |
| **Kidney Cortex** | 1,44 | **Thalamus** | 6,28 |
| **Kidney Medulla** | 0,00 | **Striatum** | 0,00 |
| **Adrenal Gland** | 0,00 | **Frontal Lobe** | 1,09 |
| **Bladder** | 0,00 | **Choroid plexus** | 0,00 |
| **Thymus** | 0,58 | **Cerebellum** | 2,20 |
| **Spleen** | 0,00 | **Circle of Willis** | 0,00 |
| **Lymphnode** | 0,00 | **Spinal Cord** | 0,00 |
| **Tonsil** | 0,00 | **DH** | 0,00 |
| **Bone Marrow** | 0,00 | **VH** | 0,00 |
| **PBMC** | 0,00 | **DRG** | 0,00 |
| **Monocytes** | 0,00 | | |

**Tab. 2**

| rat tissues | | |
|---|---|---|
| **Tissue** | Note | REU |
| **Kidney** | Sprague-Dawley rat | 0 |
| **Heart** | Sprague-Dawley rat | 0 |
| **Liver** | Sprague-Dawley rat | 0 |
| **small intestine** | Sprague-Dawley rat | 0 |
| **Lung** | Sprague-Dawley rat | 0 |
| **sk. muscle** | Sprague-Dawley rat | 0 |
| **olfactory epithel.** | Sprague-Dawley rat | 0 |
| **Ovary** | Sprague-Dawley rat | 38,3 |
| **Testis** | Sprague-Dawley rat | 81,3 |
| | | |
| **Whole brain** | Sprague-Dawley rat | 0 |
| **Cortex** | Sprague-Dawley rat | 0 |
| **hippocampus** | Sprague-Dawley rat | 0 |
| **thalamus** | Sprague-Dawley rat | 0 |
| **hypothalamus** | Sprague-Dawley rat | 0 |
| **striatum** | Sprague-Dawley rat | 0 |
| **cerebellum** | Sprague-Dawley rat | 0 |
| **astrocytes** | Sprague-Dawley rat | 0 |
| **brainstem** | Sprague-Dawley rat | 0 |
| **DH** | Sprague-Dawley rat | 0 |
| **DRG** | Sprague-Dawley rat | 0 |

**Tab. 3**

| human cell lines | |
|---|---|
| **C. line** | REU |
| **HUVEC** | 0 |
| **TF-1** | 0 |
| **Jurkat** | 0 |
| **HepG2** | 0 |
| **HUH** | 0 |
| **H4** | 0 |
| **SH-SY5Y** | 0 |
| **HEK-APP** | 0 |
| **HEK** | 0 |
| **1321N1** | 0 |
| **SKNBE** | 0 |

### REFERENCES

1. Minegish, T.; Nakamura, K.; Takakura, Y.; Ibuki, Y.; Igarashi, M. Cloning and sequencing of human FSH receptor cDNA. *Biochem. Biophys. Res. Commun.* **175:** 1125-1130, 1991.
2. Kelton, C. A.; Cheng, S. V. Y.; Nugent, N. P.; Schweickhardt, R. L.; Rosenthal, J. L.; Overton, S. A.; Wands, G. D.; Kuzeja, J. B.; Luchette, C. A.; Chappel, S. C. The cloning of the human follicle stimulating hormone receptor and its expression in COS-7, CHO, and Y-1 cells. *Molec. Cell. Endocr.* **89:** 141-151, 1992.
3. Rousseau-Merck, M. F.; Atger, M.; Loosfelt, H.; Milgrom, E.; Berger, R. The chromosomal localization of the human follicle-stimulating hormone receptor gene (FSHR) on 2p21-p16 is similar to that of the luteinizing hormone receptor gene. *Genomics* **15:** 222-224, 1993.
4. Gromoll, J.; Ried, T.; Holtgreve-Grez, H.; Nieschlag, E.; Gudermann, T.: Localization of the human FSH receptor to chromosome 2p21 using a genomic probe comprising exon 10. *J. Molec. Endocr.* **12:** 265-271, 1994.
5. Gromoll, J.; Pekel, E.; Nieschlag, E. The structure and organization of the human follicle-stimulating hormone receptor (FSHR) gene. *Genomics* **35:** 308-311, 1996.
6. Heckert, L. L.; Daley, I. J.; Griswold, M. D. Structural organization of the follicle-stimulating hormone receptor gene. *Molec. Endocr.* **6**: 70-80, 1992.
7. Sprengel R, Braun T, Nikolics K, Segaloff DL, Seeburg PH. The testicular receptor for follicle stimulating hormone: structure and functional expression of cloned cDNA. *Mol Endocrinol.* **4**: 525-530, 1990.
8. Simoni M, Gromoll J, Nieschlag E. The follicle stimulating hormone receptor. Biochemistry, molecular biology, physiology and pathophysiology. *Endocr Rev* **18**: 739-773, 1997.
9. Heckert LL, Griswold MD. The expression of the follicle-stimulating hormone receptor in spermatogenesis. *Recent Prog Horm Res* **57:** 129-48, 2002.
10. Layman LC. Mutations in the follicle-stimulating hormone-beta (FSH beta) and FSH receptor genes in mice and humans. *Semin Reprod Med* **18**(1):5-10, 2000.
11. Zeleznik AJ, Midgley AR Jr, Reichert LE Jr. Granulosa cell maturation in the rat: increased binding of human chorionic gonadotropin following treatment with follicle-stimulating hormone in vivo. *Endocrinology.* **95**(3):818-25, 1974.
12. Nimrod A, Tsafriri A, Lindner HR. In vitro induction of binding sites for hCG in rat granulosa cells by FSH. *Nature.* **267**(5612):632-3, 1977.
13. Sprengel R, Braun T, Nikolics K, Segaloff DL, Seeburg PH The testicular receptor for follicle stimulating hormone: structure and functional expression of cloned cDNA. Mol Endocrinol.;4(4):525-30, 1990.
14. Camp TA, Rahal JO, Mayo KE. Cellular localization and hormonal regulation of follicle-stimulating hormone and luteinizing hormone receptor messenger RNAs in the rat ovary. Mol Endocrinol.; 5(10):1405-17, 1991.
15. Rao, Aj, Ramachandran J. (1975). Cyclic AMP production in isolated rat seminiferous tuble cell preparations: a potential in vitro assay for follicle stimulating hormaone. Life Sci 17:411-416.
16. Kusakabe,Y., Yamaguchi,E., Tanemura,K., Kameyama,K., Chiba,N., Arai,S., Emori,Y. and Abe,K. (1998). Identification of two alpha-subunit species of GTP-binding proteins, Galpha15 and Galphaq, expressed in rat taste buds Biochim. Biophys. Acta 1403 (3), 265-272.
17. Offermanns, S., and Simon, M.I. (1995). G alpha 15 and G alpha 16 couple a wide variety of receptors phospholipase C. J. Biol. Chem. 270, 15175-15180.
18. Joseph Sambrook, David W. Russell, Joe Sambrook. Molecular Cloning: A Laboratory Manual. Cold Spring Harbor Laboratory Ed. (3rd edition), 2000.
19. Frederick M. Ausubel, Current Protocols in Molecular Biology Lippincott, Williams & Wilkins, 1988.
20. Coruzzi G, Broglie R, Edwards C, Chua NH, Tissue-specific and light-regulated expression of a pea nuclear gene encoding the small subunit of ribulose-1,5-bisphosphate carboxylase., *EMBO J.* 3(8):1671-9, 1984.
21. Engelhard EK, Kam-Morgan LN, Washburn JO, Volkman LE. The insect tracheal system: a conduit for the systemic spread of Autographa californica M nuclear polyhedrosis virus. *Proc Natl Acad Sci U.S.A.;* 91(8):3224-7, 1994.
22. Logan J, Shenk T. Adenovirus tripartite leader sequence enhances translation of mRNAs late after infection. *Proc Natl Acad Sci U.S.A.* ;81(12):3655-9, 1984.
23. Scharf KD, Materna T, Treuter E, Nover L. Heat stress promoters and transcription factors. *Results Probl Cell Differ.;* 20:125-62, 1994.
24. Wigler M, Silverstein S, Lee LS, Pellicer A, Cheng Y, Axel R. Transfer of purified herpes virus thymidine kinase gene to cultured mouse cells. *Cell.;* 11(1):223-32, 1977.
25. Lowy I, Pellicer A, Jackson JF, Sim GK, Silverstein S, Axel Isolation of transforming DNA: cloning the hamster aprt gene. *Cell.;* 22(3):817-23, 1980.
26. Wigler M, Perucho M, Kurtz D, Dana S, Pellicer A, Axel R, Silverstein S. Transformation of mammalian cells with an amplifiable dominant-acting gene. *Proc Natl Acad Sci U.S.A.;* 77(6):3567-70, 1980.
27. Colbere-Garapin F, Horodniceanu F, Kourilsky P, Garapin AC. A new dominant hybrid selective marker for higher eukaryotic cells. J Mol Biol.; 150(1):1-14, 1981.
28. Hartman SC, Mulligan RC. Two dominant-acting selectable markers for gene transfer studies in mammalian cells. Proc Natl Acad Sci U.S.A.; 85(21):8047-51, 1988.
29. Rhodes CA, Marrs KA, Murry LE. Transformation of maize by electroporation of embryos. Methods Mol Biol.; 55:121-31, 1995.
30. Lam KS. Application of combinatorial library methods in cancer research and drug discovery. Anticancer Drug Des; 12(3):145-67, 1997.
31. DeWitt SH, Kiely JS, Stankovic CJ, Schroeder MC, Cody DM, Pavia MR "Diversomers": an approach to nonpeptide, nonoligomeric chemical diversity. Proc Natl Acad Sci U.S.A.; 90(15):6909-13, 1993.
32. Erb E, Janda KD, Brenner S. Recursive deconvolution of combinatorial chemical libraries. Proc Natl Acad Sci U.S.A.; 91(24):11422-6, 1994.
33. Houghten RA, Appel JR, Blondelle SE, Cuervo JH, Dooley CT, Pinilla C. The use of synthetic peptide combinatorial libraries for the identification of bioactive peptides. Biotechniques.; 13(3):412-21, 1992.
34. Lam KS, Salmon SE, Hersh EM, Hruby VJ, Kazmierski WM, Knapp RJ. A new type of synthetic peptide library for identifying ligand-binding activity. Nature.; 354(6348):82-4, 1991.
35. Fodor SP, Rava RP, Huang XC, Pease AC, Holmes CP, Adams CL. Multiplexed biochemical assays with biological chips. Nature.; 364(6437):555-6, 1993.
36. Bush AB, Borden LA, Greene LA, Maxfield FR. Nerve growth factor potentiates bradykinin-induced calcium influx and release in PC12 cells. J Neurochem.; 57(2):562-74, 1991.
37. Higuchi R, Dollinger G, Walsh PS, Griffith R. Simultaneous amplification and detection of specific DNA sequences. *BioTechnology* **10**: 413-17, 1992.
38. Higuchi R, Fockler C, Dollinger G, Watson R. Kinetic PCR analysis: real-time monitoring of DNA amplification reactions. *BioTechnology* **11**: 1026-30, 1993.
39. Holland PM, Abramson RD, Watson R, Gelfand DH. Detection of specific polymerase chain reaction product by utilizing the 5'-3' exonuclease activity of Thermus aquaticus DNA polymerase. *Proc. Natl. Acad. Sci. U.S.A.* **88:** 7276-80, 1991.
40. Heid CA, Stevens J, Livak KJ, Williams PM. Real time quantitative PCR. *Genome Res.* **6:** 986-94, 1996.
41. Gibson UE, Heid CA, Williams PM. A novel method for real time quantitative RT-PCR. *Genome Res.* **6**: 995-1001, 1996.

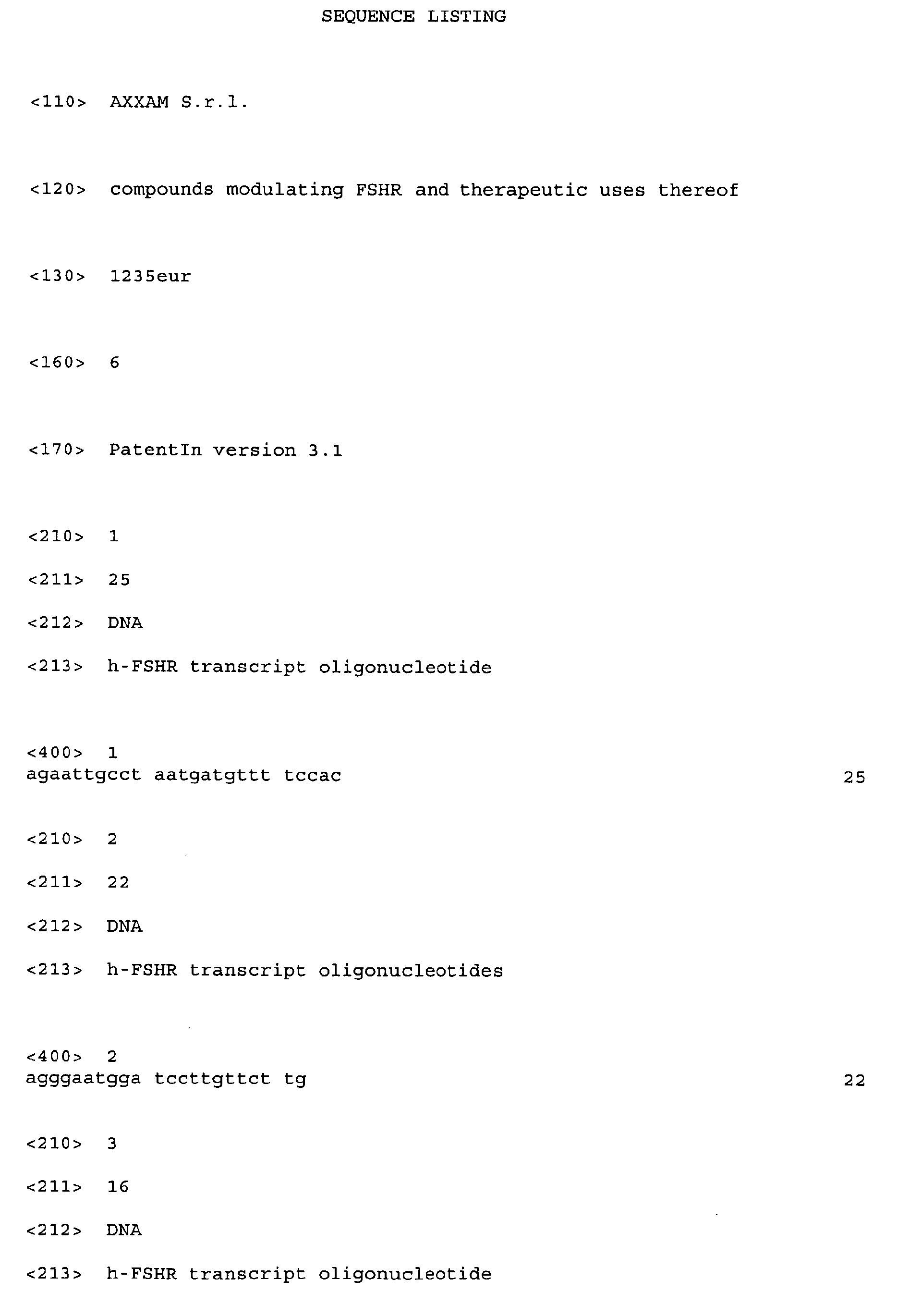

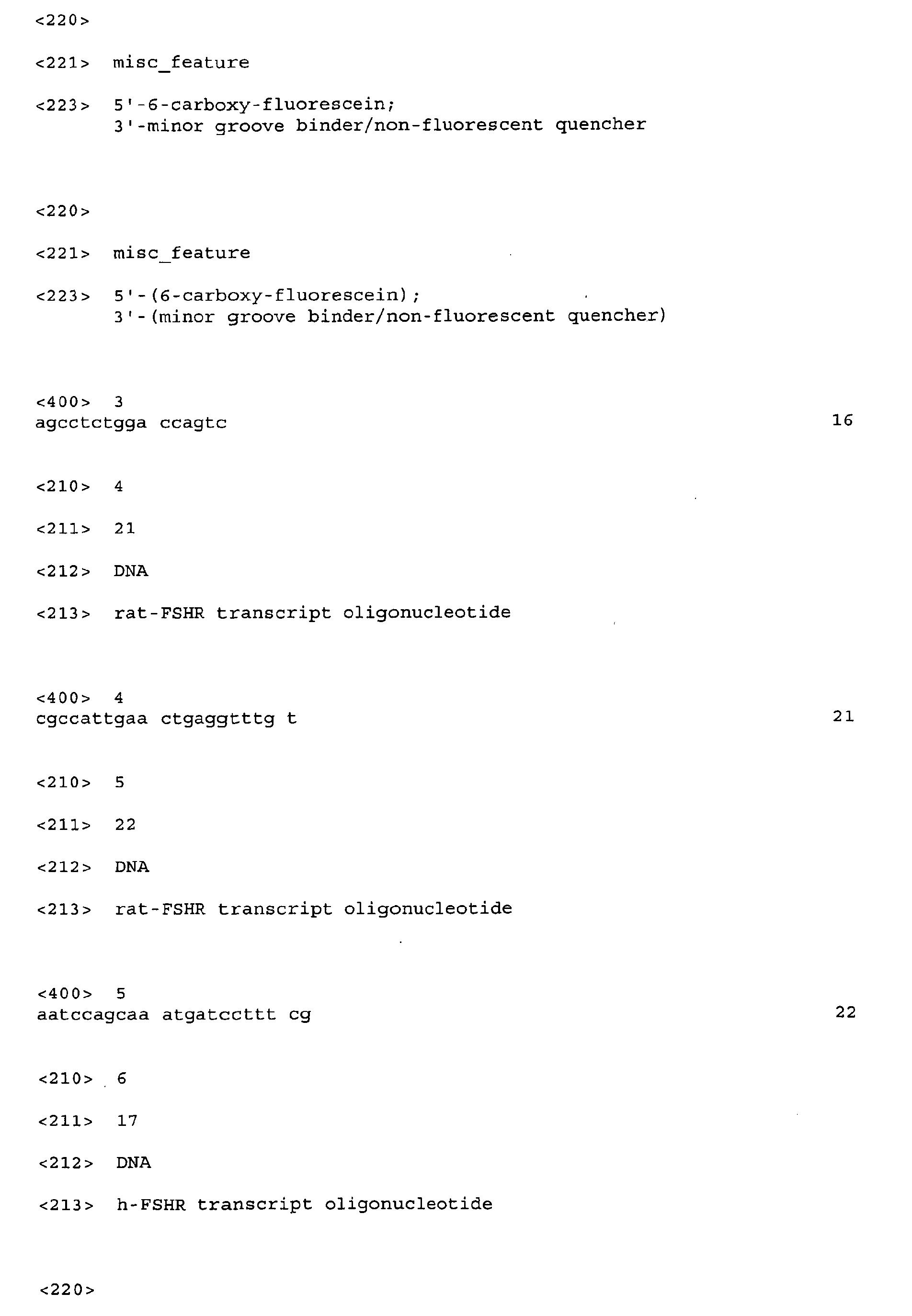

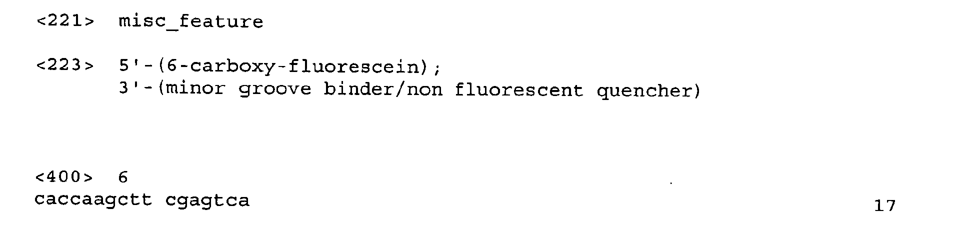

## Claims

1. Use of molecules modulating the activity of hFSH-receptor for the preparation of therapeutic agents for the treatment of diseases affecting human central and peripheral nervous system, broncus, trachea, colon, thymus, kidney, prostate, blood vessels.

2. The use according to claim 1, wherein said molecules are hFSH-receptor agonists or antagonists.

3. The use according to claim 2, wherein said molecule is FSH or an active fragment or derivative thereof.

4. The use according to claims 1-3, for the treatment of conditions of the central nervous system which involve pain, anorexia, bulimia, Parkinson's diseases, psychotic and neurological disorders, anxiety, schizophrenia, manic depression, delirium, dementia, mental retardation and dyskinesias, Huntington's disease and Touret's syndrome.

5. A method of screening compounds useful for the treatment of diseases affecting broncus and trachea, colon, thymus, kidney, prostate, blood vessels, central and peripheral nervous system, which comprises:
a) contacting a hFSHR polypeptide with a test compound;
b) detecting the binding of the polypeptide to the test compound.

6. A method according to claim 5, further comprising the selection of compounds that modulate the activity of the hFSHR polypeptide.

7. A method according to claim 6, wherein said compound is selected for its ability to increase or reduce the effects of FSHR ligands.

8. A method according to claim 7, wherein said ligand is FSH, an analogue or derivative thereof.

9. A method according to claim 7, wherein the following effects mediated by FSHR are detected:
- modulation of phospholipase C or D activity;
- cyclic AMP formation;
- mobilization of intracellular calcium.

10. A method according to claim 7, wherein compounds decreasing the functional activity of a hFSHR polypeptide by at least 10% are selected.

11. A method according to claim 10, wherein compounds decreasing the functional activity of a hFSHR polypeptide by at least 50% are selected.

12. A method according to claim 7, wherein compounds increasing the functional activity of a hFSHR polypeptide by at least 10% are selected.

13. A method according to claim 12, wherein compounds increasing the functional activity of a hFSHR polypeptide by at least 50% are selected.

14. A method according to claims 5-13, which is carried out with intact cells or with cell- or cell-membrane preparations expressing or carrying FSHR.

15. A method according to claim 14, wherein the FSHR intracellular effects are detected by means of a suitable reporter system.

16. A method according to claim 15, wherein said reporter system is a detectable reporter gene, a calcium fluorescent indicator, a luminescent protein or a radioactive reagent.

17. Use of a compound identified by the screening method of claims 5-16 for the preparation of a medicament for treating diseases involving human central and peripheral nervous system, broncus, trachea, colon, thymus, kidney, testis, prostate, ovary, blood vessels.

18. The use according to claim 17, for the treatment of conditions of the central nervous system which involve pain, anorexia, bulimia, Parkinson's diseases, psychotic and neurological disorders, anxiety, schizophrenia, manic depression, delirium, dementia, mental retardation and dyskinesias, Huntington's disease and Touret's syndrome.
